(19)

**Europäisches Patentamt**
**European Patent Office**
**Office européen des brevets**

(11) **EP 4 078 650 B1**

(12) **EUROPEAN PATENT SPECIFICATION**

(45) Date of publication and mention
of the grant of the patent:
**24.09.2025 Bulletin 2025/39**

(21) Application number: **19835814.5**

(22) Date of filing: **19.12.2019**

(51) International Patent Classification (IPC):
*H01J 49/00* (2006.01)      *G01N 33/68* (2006.01)

(52) Cooperative Patent Classification (CPC):
**H01J 49/0009; G01N 33/6851**

(86) International application number:
**PCT/IB2019/061085**

(87) International publication number:
**WO 2021/123885 (24.06.2021 Gazette 2021/25)**

(54) **CALIBRATION, METHOD OF MEASUREMENT AND MASS SPECTROMETER FOR MATERIAL QUANTITY MEASUREMENT OF INTACT MACROMOLECULES**

KALIBRIERUNG, MESSVERFAHREN UND MASSENSPEKTROMETER ZUR MATERIALMENGENMESSUNG VON INTAKTEN MAKROMOLEKÜLEN

ÉTALONNAGE, PROCÉDÉ DE MESURE ET SPECTROMÈTRE DE MASSE POUR MESURE DE QUANTITÉ DE MATÉRIAU DE MACROMOLÉCULES INTACTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.10.2022 Bulletin 2022/43**

(73) Proprietor: **Metromol SA**
**2000 Neuchâtel (CH)**

(72) Inventors:
• **TWERENBOLD, Damian**
**2017 Boudry (CH)**
• **WUNDERLI, Samuel**
**1786 Sugiez (CH)**

(74) Representative: **P&TS SA (AG, Ltd.)**
**Avenue J.-J. Rousseau 4**
**P.O. Box 2848**
**2001 Neuchâtel (CH)**

(56) References cited:
• YAO REN ET AL: "Quantification of ferritin bound iron in human serum using species-specific isotope dilution mass spectrometry", METALLOMICS, vol. 6, no. 9, 30 June 2014 (2014-06-30), GB, pages 1709 - 1717, XP055724827, ISSN: 1756-5901, DOI: 10.1039/C4MT00127C
• MATTHIAS HOPPLER ET AL: "Quantification of Ferritin-Bound Iron in Plant Samples by Isotope Tagging and Species-Specific Isotope Dilution Mass Spectrometry", ANALYTICAL CHEMISTRY, vol. 81, no. 17, 4 August 2009 (2009-08-04), pages 7368 - 7372, XP055724824, ISSN: 0003-2700, DOI: 10.1021/ac900885j
• BURKITT W I ET AL: "Toward Systeme International d'Unite-traceable protein quantification: From amino acids to proteins", ANALYTICAL BIOCHEMISTRY, ACADEMIC PRESS, AMSTERDAM, NL, vol. 376, no. 2, 15 May 2008 (2008-05-15), pages 242 - 251, XP022607165, ISSN: 0003-2697, [retrieved on 20080219], DOI: 10.1016/J.AB.2008.02.010

EP 4 078 650 B1

**Description**

The invention relates, in embodiments, to a method for calibrating a mass spectrometer for measuring intact macromolecules, such as proteins, protein complexes, viral capsules, nucleic acids, or other macromolecules or nanoparticles. The invention relates as well to a method of measuring material quantities in such a mass spectrometer and to a device therefore. The invention is particularly suitable for MALDI/TOF mass spectrometers and for detectors that can detect single molecules resolving their kinetic energy, such as superconducting junctions. Among others, the invention provides a SI-traceable measurement of the level of clinically relevant macromolecular analytes in serum, such as ferritin, antibodies, large proteins, nanoparticles, or other.

Related art

Several techniques for the measurement of macromolecules in clinical samples. Immunoassays, like the known ELISA assay, are widely used but present several limitations. In many cases, it is difficult to find an antibody with a selective affinity for a specific ligand. Another limitation lies in the fact that these methods have access only to the quasi-surface of the target molecule.
The measurement of ferritin in serum exemplifies these difficulties. The ELISA assay is not very selective for ferritin and cannot discriminate Iron-loaded ferritin from ferritin that is not filled with iron (apoferritin).
Another limitation of many biochemistry assays is that it is difficult to calibrate reliably the measurement in a SI-traceable way.
MALDI/TOF is a mass spectrometric technique that uses a laser energy absorbing matrix to create ions from large molecules with minimal fragmentation in a time-of-flight mass spectrometer. MALDI/TOF spectrometers conventionally use microchannel plates or other devices sensitive to secondary electrons for measuring the time of arrival of the molecular ions. Since the ionization probability when hitting the microchannel plate to produce secondary electrons for slow molecules is vanishingly low, their use is limited, in practice, to the molecular masses below some tens of kilodaltons.
Superconducting tunnel junctions can detect non-ionizing impacts and have been used in MALDI/TOF to detect molecules in the megadalton range and above, as disclosed by:

    1. L. D. Plath, A. Ozdemir, A. A. Aksenov and M. E. Bier, Anal. chem. 2015, 87, 8985-8993 "Determination of Iron Content and Dispersity of Intact Ferritin by Superconducting Tunnel Junction Cryodetection Mass Spectrometry"

    2. D. Twerenbold, J.-L. Vuilleumier, D. Gerber, A. Tadsen, B. van den Brandt and P. M. Gillevet, Appl. Phys. Lett. L996, 68, 3503-3505

    3. US Patent 5,640,010, D.Twerenbold "Mass spectrometer for macromolecules with cryogenic particle detectors"

In the prior art methods of species-specific isotope dilution mass spectroscopy have been described for the quantification of ferritin-bound iron:

    1. Y. Ren et al., Metallomics, 2014, 6, 1709-1717, "Quantification of ferritin bound iron in a human serum using species-specific isotope dilution mass spectrometry"

    2. M. Hoppler, et al., Analytical Chemistry, 2009, 81, 7368-7372, "Quantification of ferritin-bound iron in plant samples by isotope tagging and species-specific isotope dilution mass spectrometry

Furthermore, evidence has been provided in the prior art, that fully SI-traceable absolute protein quantification can be achieved by using double exact matching isotope dilution mass spectrometry (IDMS), provided appropriate peptides are used for quantification:

    1. W. I. Burkitt, et al., Analytical Biochemistry, 2008, 376, 242-251, "Toward Système International d'Unité-traceable protein quantification: From amino acids to proteins".

No matter the nature of the detector used to determine the time of arrival, there is a need of a method to calibrate a MALDI/TOF spectrometer in a reliable and SI-traceable way, and of the corresponding device.

Short description of the invention

An object of the present invention is the provision of a device and of a method for the SI-traceable quantitative measurement of the amount of macromolecules and for the SI-traceable calibration of the mass spectrometric detection of macromolecules.
This object is attained by the object of independent claims and in particular by a calibration method including measuring a mass distribution of intact macromolecules, having a molecular mass above 100 kDa, or above 400 kDa, or above 800 kDa, from a reference solution of the analyte with the mass spectrometer, providing an extract solution from the reference solution containing a component of the macromolecule, providing an isotope-diluted solution of the component by mixing the extract solution with an amount of an isotopically-enriched solution of the component; measuring the isotope abundance distribution of the isotope-diluted solution, measuring, based on the isotope abundance distribution of the isotope-diluted solution and on the mass distribution of intact macromolecules, a calibration coefficient linking the amount or the concentration (relative to volume) or content (relative to mass) of the analyte to integrated area of a region in the mass distribution.

The object of the invention includes also a measuring method and a calibrated mass spectrometer.

Dependent claims introduce important and advantageous features that are not, however, essential for the invention, notably: the use of a MALDI/TOF spectrometer, possibly with a cryogenic detector sensitive to the kinetic energy of the individual macromolecules, the use of a double isotope-dilution method to measure the amount of substance content or concentration of the component or the isotope abundance ratio in the isotopically-enriched solution, the nature of the analyte and its component that may be is iron-loaded ferritin, respectively iron, or an antibody, respectively sulphur, the use of a recombinant pure substance for the loaded under controlled conditions to provide the iron-loaded ferritin reference.

Figures

Embodiment(s) of the invention will now be disclosed with reference to the figures that illustrate schematically:

- figure 1 a method according to an aspect of the invention.

- figure 2, the production of the reference substance, exemplified by the embodiment of the iron-loaded ferritin,

- figure 3, the measurement of the mass distribution of intact macromolecules, using the example of the iron-loaded ferritin,

- figure 4, the SI-traceable measurement of the amount of analyte, illustrated by the embodiment of the iron-loaded ferritins using a double isotopic dilution method.

Detailed description

The present disclosure will describe in detail embodiments of the present invention that concern the measurement of the material quantity of iron-loaded ferritin, with the of the amount of substance quantity of iron, as a component of the target analyte, by isotope dilution. Although this is an important and advantageous use case, the invention is not limited to this application and includes other variants.

A possible variant of the invention is the calibration of a mass spectrometer for the measurement of antibodies. In this case, iron is not present in the target analyte, and another component is selected, for example sulphur.

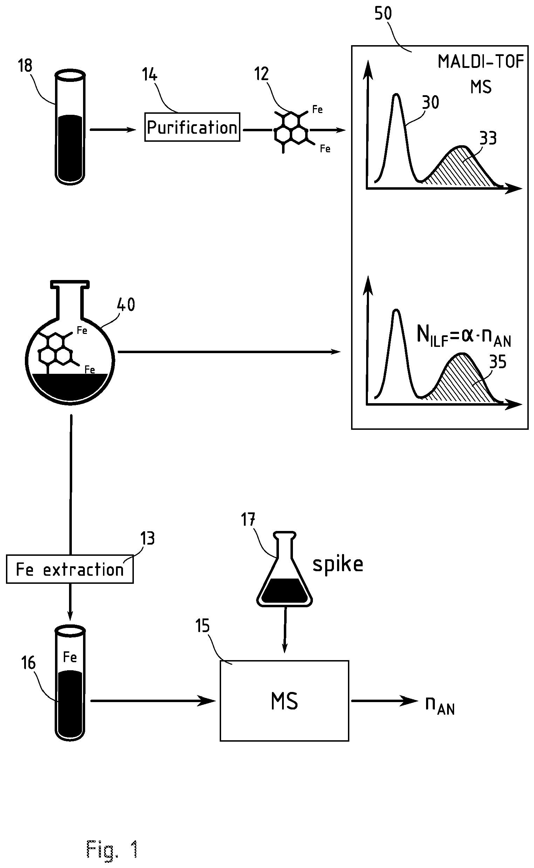

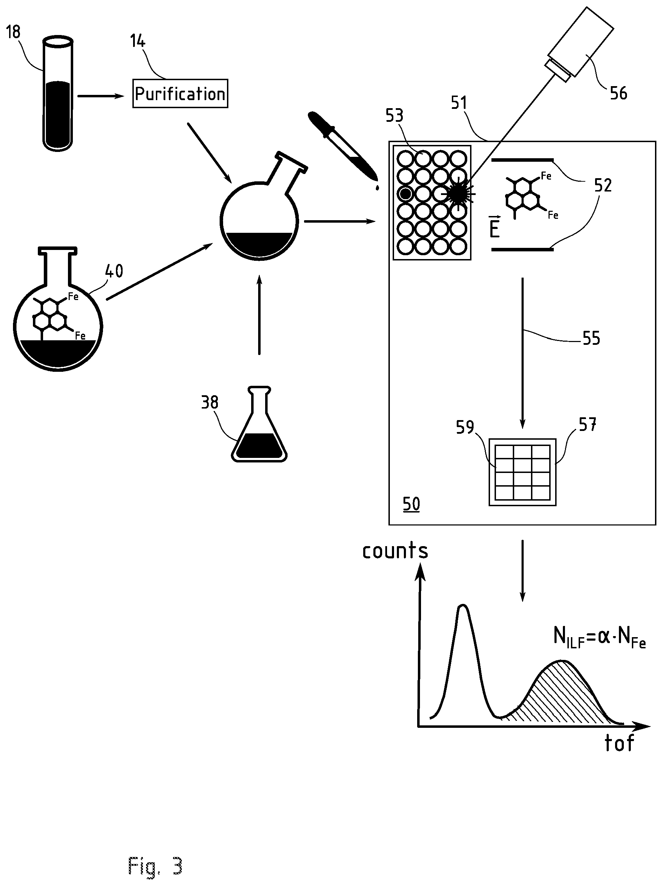

As shown in figures 1 and 3. A biological sample material 18, for example serum, is analysed to measure the material quantity of iron-loaded ferritin (ILF for brevity). After an optional purification 14 the sample is mixed with a suitable matrix 38 (good results have been obtained with a dried matrix of sinapinic acid or picolinic acid dissolved in a mixture of water, ethanol, and trifluoroacetic acid, but other choices are possible), dried on a target plate 53, and inserted in the vacuum chamber 51 of a MALDI-TOF spectrometer 50. The target plate 53 may have a plurality of wells for different samples that can be analysed in succession and may include the biologic sample 18 as well as reference solutions and isotope-diluted references, as it will be disclosed in the following.

In the spectrometer 50, a laser 56, for example a nitrogen UV laser, is aimed at the wells of the target plate where it causes the macromolecules in the sample to be desorbed and ionized. The free molecules are accelerated by high voltage electrodes 52 and travel along the free flight zone to the detector 59 where they are detected. Preferably, the detector 59 comprises an array of superconducting Josephson junctions in a cryostat 57 that can detect individual molecule impacts and measure their kinetic energy and their charge, even if the impact speed is too low for the emission of secondary electrons. The time elapsed between the laser pulse and the impact measured at the detector 59 is a measure of the molecule speed, from which the mass of the molecular ion can be computed.

Other MS configurations, for example including reflectrons, are possible and included in the scope of the present invention.

After a sufficient number of molecules have been detected and their masses signals accumulated, the mass spectrum (visible in figure 1) presents a region of interest 33 whose area correspond to the total number of molecules detected, for example iron-loaded ferritin, and other features not necessarily related to the analyte in study, such as the peak 30 that corresponds to apoferritin. Other features that may appear on the spectrum are fragments, or subunit of the analyte, or ions with multiple charges. These features can be discriminated based on the mass (based on the time of flight) and in the case of multiply-charged ions, from their kinetic energy.

Besides the clinical sample 18, the invention foresees that a reference solution 40 containing the macromolecule analyte is measured and its mass spectrum is taken. This yields a second mass spectrum with similar features.

According to the invention, the amount of analyte of the reference solution 40 is measured by isotope dilution on a fragment of the macromolecule of low mass, possibly an elemental component, by a second mass spectrometer adapted for the measure of isotope abundance ratios. Thanks to this measurement, a calibration factor $\alpha$ linking the integral of the region of interest 33 with the material amount of the target analyte in the initial solution can be determined.

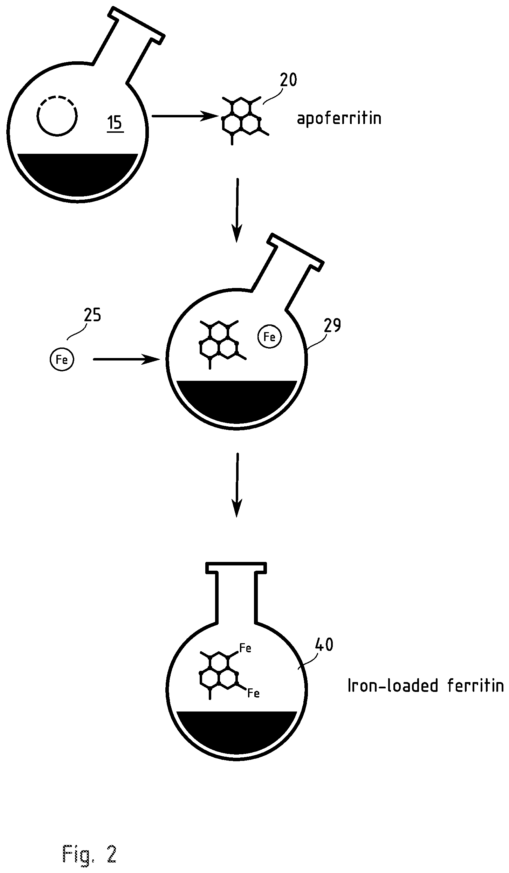

Advantageously, as depicted in figure 2, the reference solution 40 is prepared from a pure recombinant macromolecule 20, (in this embodiment, apoferritin) produced by a suitable genetically manipulated host 15. The apoferritin is loaded by iron ions 25 under controlled conditions 29.

Returning to figure 1, a measured or known amount of reference solution 40 is taken. Extraction (separation, digestion) step 13 provides a solution of an element that can be measured by isotope dilution.

In the ferritin measurement embodiment, it is advantageous to choose iron as element, because iron has several suitable stable isotopes ($^{56}$Fe and $^{57}$Fe notably) to which isotope dilution can be applied, and because the amount of iron bound to serum ferritin is clinically significant. The extract 16 may contain ferrous ($Fe^{2+}$) or ferric ($Fe^{3+}$) ions, or another form of elemental or molecular iron in any oxidation state preferably low molecular species or single ions.

In other embodiments, where one search a calibration of a mass spectrometer for another macromolecule, iron may be replaced by another suitable component. Sulphur, for example, may be extracted and used in lieu of iron in the determination of the material quantity of antibodies or other proteins, large biomolecules or similar.

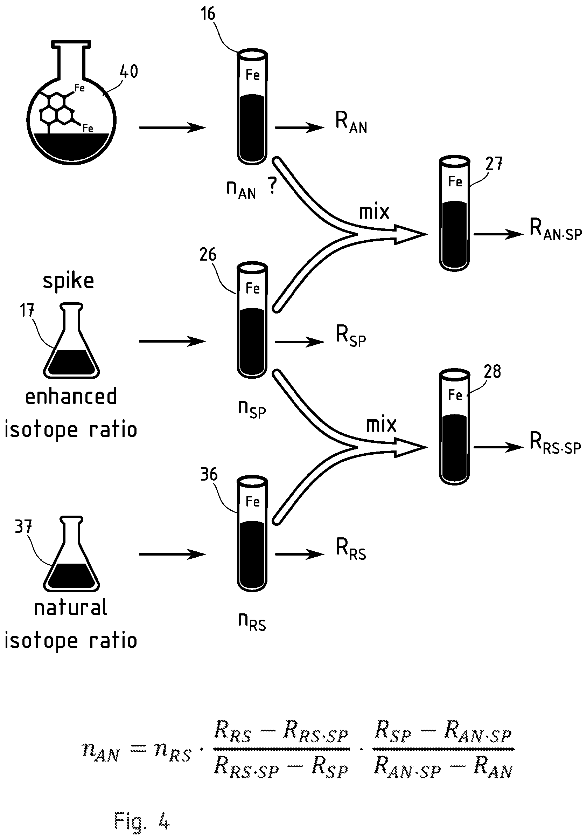

Isotope dilution allows the measurement of the material number quantity of iron $n_{Fe}$ in the reference solution 4. Preferably, as shown in figure 4, a double isotope dilution mass spectrometry (reverse IDMS) is used. While reverse IDMS is known in other contexts, it has never been applied to the calibration of mass spectrometers for macromolecules as claimed. The reference solution 40 with isotopic abundance ratio $R_{AN}$ is mixed with an isotopically enhanced solution 17 (the "spike" solution) with isotopic ratio $R_{SP}$ and the isotopic abundance ratio $R_{AN \cdot SP}$ of the mixture 27 is determined by a high-resolution mass spectrometer specialized for measuring masses close to 56-57 u.

Another mixture 28 is produced by mixing the isotopically enhanced solution 17 with a reference solution of the component 37, precisely measured, with isotopic abundance ratio $R_{RS}$, and the isotopic abundance ratio $R_{RS \cdot SP}$ of the mixture 28 is measured in the same way as that of mixture 27. The number quantity of iron in the reference solution 40 is then given by

$$n_{AN} = n_{RS} \cdot \frac{R_{RS} - R_{RS \cdot SP}}{R_{RS \cdot SP} - R_{SP}} \cdot \frac{R_{SP} - R_{AN \cdot SP}}{R_{AN \cdot SP} - R_{AN}}$$

Since the isotope abundance ratios $R_{RS}$ and $R_{AN}$ are both equal to the natural isotopic abundance ratio of iron, the equation above gives the number quantity or amount of iron in the reference solution eliminating the need to measure the quantity of the isotopically-enhanced solution 17.

From $n_{AN}$, one derives the proportionality coefficient $\alpha$ between the amount of the analyte in the reference solution 40 and the integral 35 of the mass distribution of the reference solution in the mass-region corresponding to iron-loaded ferritin. Since the amount of iron is bound to the ferritin in both cases, the same coefficient links the integral 33 in the mass distribution of the sample 18 with the amount of analyte in the same. The MALDI-TOF mass spectrometer 50 is calibrated for the analyte by a calibration coefficient $\alpha$.

Reference numbers used in the drawings

| 12 | macromolecule analyte, ferritin |
| 13 | extraction of a component |
| 14 | purification |
| 15 | apoferritin produced |
| 16 | extract solution |
| 17 | isotope-diluted solution |
| 18 | biological sample, serum |
| 20 | recombinant apoferritin |
| 25 | macromolecule component, iron |
| 26 | isotope-enhances solution |
| 27 | first diluted solution |
| 28 | second diluted solution |
| 29 | controlled loading of apoferritin |
| 30 | apoferritin peak |
| 33 | iron-loaded ferritin peak |
| 35 | iron-loaded peak in the reference solution |
| 36 | natural-isotope reference solution |
| 37 | natural-isotope reference solution |
| 38 | matrix, e.g. sinapinic acid |
| 40 | reference solution |
| 50 | MALDI/TOF mass spectrometer |
| | |
| 51 | vacuum chamber |
| 52 | high-voltage electrodes |
| 53 | sample target |
| 55 | free flight |
| 56 | UV laser |
| 57 | cryostat |
| 59 | superconducting molecule detectors |

**Claims**

1. Method of calibrating a mass spectrometer (50) for macromolecules for measuring the material quantity of an analyte, wherein the analyte is a macromolecule having a molecular mass above 100 kDa, or above 400 kDa, or above 800 kDa , the method including

    • measuring a mass distribution of intact macromolecules from a reference solution (40) of the analyte with the mass spectrometer (50),
    • providing an extract solution (16) from the reference solution (40) containing a component of the macromolecule,
    • providing an isotope-diluted solution (27) of the component by mixing the extract solution (16) with an amount (26) of an isotopically-enriched solution of the component (17),

• measuring the isotope abundance ratio ($R_{AN \cdot SP}$) of the isotope-diluted solution, and

• determining, based on the isotope abundance ratio ($R_{AN \cdot SP}$) of the isotope-diluted solution and on the molecular mass distribution of intact macromolecules, a calibration coefficient ($\alpha$) linking the amount of substance, content or the concentration of analyte to the integrated area of a region in the mass distribution.

2. The method of the preceding claim, wherein the calibration is SI traceable.

3. The method of any one of the preceding claims, wherein the mass-spectrometer is a MALDI/TOF spectrometer.

4. The method of the preceding claim, wherein the spectrometer includes a cryogenic detector (59) sensitive to the kinetic energy of the individual macromolecules impinging on the detector (59).

5. The method of any one of the preceding claims, including measuring an amount of the component or number quantity ($n_{SP}$) or an isotope abundance ratio ($R_{SP}$) in the isotopically-enriched solution of the component (16) by measuring the isotope abundance ratio ($R_{RS \cdot SP}$) of a second diluted solution (28) obtained by mixing the isotopically-enriched solution of the component (17) with an amount (36) of a standard solution of the component (37) having a natural isotope abundance ratio.

6. The method of any one of the preceding claims, wherein the analyte is iron-loaded ferritin, and the component is iron.

7. The method of the preceding claim, wherein the reference solution contains recombinant apoferritin (20) which is loaded under controlled conditions (29) with iron (25).

8. The method of any one of claims 1 to 5, wherein the analyte is a protein or an antibody, and the component is sulphur or a small molecule comprising sulphur or another suitable element or marker.

9. A method of measuring the amount of an analyte in a biological sample, wherein the analyte is a macromolecule in a mass spectrometer (50), the method comprising calibrating the mass spectrometer for the analyte by the method of any one of the preceding claims prior to said measuring.

10. The method of the preceding claim, wherein the biological sample (18) consists of serum or biological liquid or biological tissue.

11. Device for measuring the amount of an analyte in a biological sample, wherein the analyte is a macromolecule and the device includes a MALDI-TOF spectrometer and means configured to calibrate the mass spectrometer for the analyte using the method of any one of claims 1-7.

12. The device of the preceding claim, comprising a cryogenic detector (59) sensitive to the kinetic energy of the individual macromolecules impinging on the detector (59).

**Patentansprüche**

1. Verfahren zum Kalibrieren eines Massenspektrometers (50) für Makromoleküle zum Messen der Materialmenge eines Analyten, worin der Analyt ein Makromolekül mit einer Molekülmasse über 100 kDa oder über 400 kDa oder über 800 kDa ist, wobei das Verfahren umfasst:

Messen einer Massenverteilung intakter Makromoleküle aus einer Referenzlösung (40) des Analyten mit dem Massenspektrometer (50);
Bereitstellen einer Extraktlösung (16) aus der Referenzlösung (40), die eine Komponente des Makromoleküls enthält,
Bereitstellen einer isotopenverdünnten Lösung (27) der Komponente durch Mischen der Extraktlösung (16) mit einer Menge (26) einer isotopenangereicherten Lösung der Komponente (17);
Messung des Isotopenhäufigkeitsverhältnisses ($R_{AN\ SP}$) der isotopenverdünnten Lösung, und auf Grund des Isotopenhäufigkeitsverhältnisses ($R_{AN\ SP}$) der isotopenverdünnten Lösung und der Molekülmassenverteilung intakter Makromoleküle, Bestimmen eines Kalibrierungskoeffizients ($\alpha$), der die Substanzmenge, den Gehalt oder die Konzentration des Analyten mit der integrierten Fläche eines Bereichs in der Massenverteilung verknüpft.

2. Verfahren gemäss dem vorhergehenden Anspruch, worin die Kalibrierung SI-rückführbar ist.

3. Verfahren gemäss irgendeinem der vorhergehenden Ansprüche, worin das Massenspektrometer ein MALDI/TOF-Spektrometer ist.

4. Verfahren gemäss dem vorhergehenden Anspruch, worin das Spektrometer einen kryogenen Detektor (59) umfasst, welcher auf die kinetische Energie der einzelnen Makromoleküle reagiert, die auf den Detektor (59) auftreffen.

**5.** Verfahren gemäss irgendeinem der vorhergehenden Ansprüche, umfassend das Messen einer Menge der Komponente oder einer Anzahlmenge ($n_{SP}$) oder eines Isotopenhäufigkeitsverhältnisses ($R_{SP}$) der isotopenangereicherten Lösung der Komponente (16) durch Messen des Isotopenhäufigkeitsverhältnisses ($R_{RS\ SP}$) einer zweiten verdünnten Lösung (28), welche durch Mischen der isotopenangereicherten Lösung der Komponente (17) mit einer Menge (36) einer Standardlösung der Komponente (37) mit einem natürlichen Isotopenhäufigkeitsverhältnis erhalten wird.

**6.** Verfahren gemäss irgendeinem der vorhergehenden Ansprüche, worin der Analyt eisenhaltiges Ferritin und die Komponente Eisen ist.

**7.** Verfahren gemäss dem vorhergehenden Anspruch, worin die Referenzlösung rekombinantes Apoferritin (20) enthält, welches unter kontrollierten Bedingungen (29) mit Eisen (25) beladen wird.

**8.** Verfahren gemäss irgendeinem der Ansprüche 1 bis 5, worin der Analyt ein Protein oder ein Antikörper ist, und die Komponente Schwefel oder ein kleines Molekül ist, das Schwefel oder ein anderes geeignetes Element oder einen anderen geeigneten Marker enthält.

**9.** Verfahren zum Messen der Menge eines Analyten in einer biologischen Probe, worin der Analyt ein Makromolekül in einem Massenspektrometer (50) ist, wobei das Verfahren die Kalibrierung des Massenspektrometers für den Analyten durch das Verfahren gemäss irgendeinem der vorhergehenden Ansprüche vor der besagten Messung umfasst.

**10.** Verfahren gemäss dem vorhergehenden Anspruch, worin die biologische Probe (18) aus Serum oder biologischer Flüssigkeit oder biologischem Gewebe besteht.

**11.** Gerät zum Messen der Menge eines Analyten in einer biologischen Probe, worin der Analyt ein Makromolekül ist, und das Gerät ein MALDI-TOF-Spektrometer umfasst sowie Mittel, welche dazu konfiguriert sind, das Massenspektrometer für den Analyten unter Anwendung des Verfahrens gemäss irgendeinem der Ansprüche 1-7 zu kalibrieren.

**12.** Vorrichtung gemäss dem vorhergehenden Anspruch, umfassend einen kryogenen Detektor (59), welcher auf die kinetische Energie der einzelnen Makromoleküle reagiert, die auf den Detektor (59) auftreffen.

**Revendications**

**1.** Procédé de calibration d'un spectromètre de masse (50) pour des macromolécules pour macromolécules pour mesurer la quantité de matière d'un analyte, dans lequel l'analyte est une macromolécule avec une masse moléculaire au-dessus de 100 kDa, ou au-dessus de 400 kDa, ou au-dessus de 800 kDa, le procédé comprenant

  • mesurer une distribution de masses de macromolécules intactes depuis une solution de référence (40) de l'analyte avec le spectromètre de masse (50),
  • fournir une solution d'extraction (16) de la solution de référence (40) contenant une composante de la macromolécule,
  • fournir une solution de dilution isotopique (27) de la composante en mélangeant la solution d'extraction (16) avec une quantité (26) d'une solution enrichie isotopiquement de la composante (17),
  • mesurer le rapport d'abondance isotopique ($R_{AN \cdot SP}$) de la solution de dilution isotopique, et
  • déterminer, se basant sur le rapport d'abondance isotopique ($R_{AN \cdot SP}$) de la solution de dilution isotopique et sur la distribution de masses des macromolécules intactes, un coefficient de calibration ($\alpha$) liant la quantité de matière, ou le contenu, ou la concentration d'analyte à une aire intégrée d'une région dans la distribution de masses.

**2.** Le procédé de la revendication précédente, dans lequel la calibration est traçable au SI.

**3.** Le procédé de l'une quelconque des revendications précédentes, dans lequel le spectromètre de masse est un spectromètre MALDI/TOF.

**4.** Le procédé de la revendication précédente, dans lequel le spectromètre comprend un détecteur cryogénique (59) sensitif à l'énergie cinétique des macromolécules individuelles qui frappent sur le détecteur (59).

**5.** Le procédé de l'une quelconque des revendications précédentes, comprenant la mesure d'une quantité de la composante ou de la quantité ou de la quantité numérique ($n_{SP}$) ou d'un rapport de abondance isotopique ($R_{SP}$) dans la solution enrichie isotopiquement de la composante (16) en mesurant le rapport d'abondance isotopique ($R_{RS \cdot SP}$) d'une seconde solution de dilution (28) obtenue en mélangeant la solution enrichie isotopiquement de la composante (17) avec une quantité (36) d'une solution standard de la composante (37) avec un rapport d'abondance isotopique naturel.

**6.** Le procédé de l'une quelconque des revendications précédentes, dans lequel l'analyte est ferritine chargée en fer, et la composante est le fer.

**7.** Le procédé de la revendication précédente, dans lequel la solution de référence contient de l'apoferritine recombinante (20) qui est chargée en fer (25) sous conditions contrôlées (29).

**8.** Le procédé de l'une quelconque des revendications de 1 à 5, dans lequel l'analyte est une protéine ou un anticorps, et la composante est le soufre ou une petite molécule comprenant du soufre ou un autre élément ou marqueur convenable.

**9.** Un procédé de mesure de la quantité d'un analyte dans un échantillon biologique, dans lequel l'analyte est une macromolécule, dans un spectromètre de masse (50), le procédé comprenant la calibration du spectromètre de masse (50) pour l'analyte avec le procédé de l'une quelconque des revendications précédentes avant ladite mesure.

**10.** Le procédé de la revendication précédente, dans lequel l'échantillon biologique (18) consiste en sérum u en un fluide biologique ou en un tissu biologique.

**11.** Dispositif pour la mesure de la quantité d'un analyte dans un échantillon biologique dans lequel l'analyte est une macromolécule et le dispositif comprend un spectromètre MALDI-TOF et des moyens configurés pour calibrer le spectromètre de masse pour l'analyte en utilisant le procédé de l'une quelconque des revendications 1-7.

**12.** Le dispositif de la revendication précédente comprenant un détecteur cryogénique (59) sensitif à l'énergie cinétique des molécules individuelles qui frappent le détecteur (59).

Fig. 1

20

apoferritin

25

29

40

Iron-loaded ferritin

Fig. 2

Fig. 3

$$n_{AN} = n_{RS} \cdot \frac{R_{RS} - R_{RS \cdot SP}}{R_{RS \cdot SP} - R_{SP}} \cdot \frac{R_{SP} - R_{AN \cdot SP}}{R_{AN \cdot SP} - R_{AN}}$$

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5640010 A, D.Twerenbold

**Non-patent literature cited in the description**

- **L. D. PLATH** ; **A. OZDEMIR** ; **A. A. AKSENOV** ; **M. E. BIER**. Determination of Iron Content and Dispersity of Intact Ferritin by Superconducting Tunnel Junction Cryodetection Mass Spectrometry. *Anal. chem.*, 2015, vol. 87, 8985-8993
- **D. TWERENBOLD** ; **J.-L. VUILLEUMIER** ; **D. GERBER** ; **A. TADSEN** ; **B. VAN DEN BRANDT** ; **P. M. GILLEVET**. *Appl. Phys. Lett. L996*, vol. 68, 3503-3505
- **Y. REN et al.** Quantification of ferritin bound iron in a human serum using species-specific isotope dilution mass spectrometry. *Metallomics*, 2014, vol. 6, 1709-1717
- **M. HOPPLER et al.** Quantification of ferritin-bound iron in plant samples by isotope tagging and species-specific isotope dilution mass spectrometry. *Analytical Chemistry*, 2009, vol. 81, 7368-7372
- **W. I. BURKITT et al.** Toward Système International d'Unité-traceable protein quantification: From amino acids to proteins. *Analytical Biochemistry*, 2008, vol. 376, 242-251